# FASCICULE DE BREVET EUROPEEN

(11) **EP 0 575 273 B1**
(45) Date de publication et mention de la délivrance du brevet: **03.12.1997**
(21) Numéro de dépôt: 93420255.7
(22) Date de dépôt: 17.06.1993
(51) Int. Cl.: C08H 1/06, A61L 15/32, A61L 25/00, A61L 27/00

(54) **Dérivés réticulables de collagène, leur procédé d'obtention et leur application à la préparation de biomatériaux**
Vernetzbare Kollagenderivate, Verfahren zu ihrer Herstellung und ihre Verwendung zur Herstellung von Biomaterialien
Crosslinkable derivatives of collagen, process for obtaining the same and their use in the preparation of biomaterials

(30) Priorité: 18.06.1992 FR 9207692
(43) Date de publication de la demande: 22.12.1993
(73) Titulaire: FLAMEL TECHNOLOGIES, Société Anonyme, F-69693 Venissieux Cédex (FR)
(72) Inventeur: Gagnieu, Christian, F-69680 Chassieu (FR)
(74) Mandataire: Ropital-Bonvarlet, Claude

(56) Documents cités:
- EP-A- 0 049 469
- EP-A- 0 191 994
- EP-A- 0 330 389
- US-A- 4 294 241
- ANGEW. CHEM. vol. 74, no. 22, 1962, page 904 F. SCHADE & H. ZAHN 'Einbau von Cystin-Brücken in Kollagen'

## Description

La présente invention est relative à de nouveaux dérivés réticulables de collagène susceptibles d'être utilisés dans la préparation de biomatériaux, à partir desquels des produits applicables, notamment en médecine, et plus particulièrement en chirurgie, ou en cosmétologie, peuvent être obtenus.

Parmi ces produits, on peut citer les tissus ou les organes artificiels, tels que la peau artificielle, les prothèses ou implants osseux, ligamentaires, cardio-vasculaires, intra-oculaires..., ou bien encore les systèmes d'encapsulation (implants, microsphères, microcapsules) permettant la libération contrôlée de principes actifs in vivo et de la bioencapsulation.

A titre d'exemple, on peut également évoquer les accessoires médicaux, tels que les fils de sutures ou les revêtements de biocompatibilisation d'articles médicaux implantables, ainsi que les solutions ou suspensions de collagène injectables, utilisées pour le comblement et la reconstruction tissulaire.

Pour chacune des applications biomédicales précitées, il est indispensable d'obtenir pour le collagène réticulé certaines propriétés physicochimiques, mécaniques ou biologiques de façons reproductibles et contrôlées. Seules la parfaite maîtrise des modifications chimiques du collagène, la large gamme des produits synthétisés selon l'invention et la bonne adaptabilité des procédés de réticulation qui en découlent permettent de répondre de façon satisfaisante à la plupart des contraintes apparaîssant lors de l'élaboration du cahier des charges d'une application donnée.

L'invention concerne, également, un procédé d'obtention de ces nouveaux dérivés réticulables de collagène, ainsi que de nouveaux produits intermédiaires intervenant dans le susdit procédé, et enfin, le collagène réticulé issu du collagène réticulable considéré.

Le domaine de l'invention est celui des matériaux biocompatibles à base de collagène, utiles comme matière première pour la réalisation d'articles destinés à être mis en contact avec ou implantés dans le corps humain ou animal, et capables de s'assimiler au mieux aux matériaux biologiques, notamment sur le plan mécanique, de manière à pouvoir les remplacer. L'application visée est essentiellement la médecine humaine ou vétérinaire.

Le collagène est une protéine connue, présente à tous les niveaux de l'organisation des tissus animaux : il s'agit de la principale protéine de la peau et du tissu conjonctif. Par nature, il possède des caractéristiques biochimiques et physico-chimiques relativement bien adaptées au milieu vivant.

EP-A-0 049 469 divulgue la réaction du collagène avec un ester activé de cystine. EP-A-0 191 944 décrit la réaction de l'anhydre succinique avec les groupes NH₂ libres du collagène.

Au sens de la présente invention, le terme collagène désigne tout peptide de nature collagènique, dont notamment la gélatine.

Différentes qualités de collagène d'origine animale ou humaine sont actuellement commercialisées dans le monde, essentiellement pour l'élaboration de biomatériaux ou de produits cosmétiques.

Dans les applications répandues à l'heure actuelle, on se satisfait des propriétés des différentes qualités disponibles.

Ainsi, parmi ces collagènes on trouve d'excellents supports d'adhésion, de multiplication et de croissance cellulaire, appréciés pour la réalisation de milieux de culture cellulaire.

On tire également profit de leur hydrophilie, de leur faible immunogénicité et de leur caractère hémostatique.

Les propriétés mécaniques des collagènes natifs sont acceptables pour un certain nombre d'utilisations.

Cependant, force est de reconnaître que dans le domaine des articles médicaux implantables, tels que les implants et les prothèses, les collagènes natifs du marché souffrent d'importantes lacunes quant à leur résistance mécanique et à leur résistance à la protéolyse.

En effet, l'introduction de ces corps étrangers, que constituent les implants et les prothèses, dans un organisme vivant induit des phénomènes de rejet qui se traduisent, notamment, par des réactions inflammatoires provoquant, entre autre, la production de collagénase, qui hydrolyse le collagène. Il en résulte, pour le moins, une altération du comportement mécanique du greffon à base de collagène.

On sait que la réticulation permet d'améliorer les propriétés mécaniques du collagène. Elle confère aux fibres de collagène une très grande résistance à la traction et à la déchirure, grâce aux nombreuses liaisons covalentes qu'elle crée entre les chaînes de collagène.

Sur la base de cette connaissance scientifique, de nombreuses études ont été entreprises pour développer les possibilités de réticulation artificielle du collagène.

Il est ainsi apparu trois grands types de techniques de réticulation de cette protéine.

Le premier type de technique est la réticulation à l'aide d'agent pontant dans laquelle des molécules exogènes, le plus souvent bifonctionnelles, sont greffées pour permettre l'établissement des liaisons. Les réactifs les plus fréquemment employés sont :
- Les mono- et di-aldéhydes tels que le formaldéhyde (engendrant des ponts méthylènes), le malonaldéhyde et surtout le glutaraldéhyde (réticulant par liaisons imines et aldols). Les principaux problèmes sont dus à leurs extrémités -CHO qui sont irritantes et à l'autopolymérisation des di-aldéhydes qui les rend cytotoxiques.
- Les composés dicarboxyliques qui sont, à ce jour, essentiellement employés soit pour modifier le collagène, soit pour le tannage des peaux et qui agissent par liaisons amides voire esters.
- Les diamines, telles que l'hexaméthylène diamine, qui agissent uniquement par liaisons amides.
- Les diisocyanates, dont l'hexamethylène diisocyanate qui est utilisé pour la réticulation par liaisons amides.
- Les disulfonyl-chlorures qui établissent des liaisons intra et inter-caténaires.

Suivant un second type de technique, on procède à la création d'un réseau par liaison covalente entre les molécules de collagène, et ce sans greffage de composés exogènes.

Les principales méthodes employées sont :
- L'irradiation (rayonnement ultra-violet ou gamma) qui produit à la fois un certain nombre de désaminations oxydatives, permettant des réticulations par liaisons imines et aldols, ainsi que des radicaux libres très réactifs pouvant créer des pontages covalents. Une telle méthode présente l'inconvénient de provoquer la réticulation du collagène seulement dans une étroite zone, pour des basses énergies, tandis que pour des énergies supérieures, elle entraîne des hydrolyses ou des dénaturations très préjudiciables au produit.
- La déshydratation (poussée : plus de 100° C, sous vide) qui conduit à la formation de liaisons amides et esters, ainsi que de lysino-alanines intra et intermoléculaires. Parmi les réactifs employés, on peut citer les carbodiimides, tels que le cyanamide ou le dicyclohexylcarbodiimide. Ce mode de réticulation en est encore au stade expérimental.
- La réticulation enzymatique par des protéines mimant l'effet de la lysyloxydase (enzyme responsable de la réticulation naturelle). Cela reste pour le moment à l'étude.
- L'oxydo-réduction qui induit une désamination oxydative des extrémités aminées qui deviennent aldéhydiques. On utilise essentiellement des cations métalliques (Cu²⁺, Fe²⁺, Al³⁺) associés à des cofacteurs (Ascorbate, Pyridoxal-5P), ainsi que des sulfites ou des nitrites. Cette méthode est très utilisée pour le tannage du cuir.
- L'activation fonctionnelle, en particulier des carboxyles qui peuvent fournir des azides d'acides ayant une réactivité très sélective vis-à-vis des extrémités -NH₂ et conduisant à la formation d'une liaison amide. Divers biomatériaux peuvent être réalisés ainsi.

Le troisième type de technique est la réticulation par copolymérisation. Elle consiste à réunir, par des liaisons covalentes, le collagène à un autre polymère pour donner des conformations plus ou moins enchevêtrées. Les polymères les plus souvent associés au collagène sont :
- les dérivés d'acrylique, dont la toxicité est souvent rédhibitoire pour les applications en médecine humaine du type implant,
- les mélanges acrylonitrile-styréne avec lesquels il n'a jusqu'alors pas été possible de dépasser le stade du laboratoire,
- les polyuréthannes surtout utilisés dans le renforcement des cuirs tannés,
- les polyalcools,
- et les silicones.

Les liaisons impliquées dans la copolymérisation sont très diverses et dépendent des groupements mis en jeu par chaque polymère.

Toutes ces techniques, qu'elles soient de nature physique ou chimique, possèdent de nombreux inconvénients.

Tout d'abord, concernant les réticulations chimiques, elles donnent lieu à des résidus toxiques dans le collagène réticulé. Les résidus peuvent être sous forme de réactifs non consommés ou de fonctions réactives libres, issues de réactifs bifonctionnels qui n'ont réagi que par une seule extrémité.

S'agissant des réticulations physiques, elles sont toutes de mise en oeuvre délicate et manquent de reproductibilité.

De manière générale, ces deux types de réticulation entraînent une perte partielle ou totale de l'affinité des cellules tissulaires pour le collagène modifié.

En outre, elles ne peuvent pas être mises en oeuvre pour l'obtention d'articles moulés, à partir de solutions de collagène. En effet, aucune d'elles ne permet de maîtriser la cinétique de réticulation, pas plus que le taux de réticulation.

Dans de telles conditions aléatoires, il n'est pas possible d'envisager des fabrications industrielles simples, économiques et conduisant à des produits de qualité mécanique adaptée aux applications visées.

C'est la raison pour laquelle, dans la grande majorité des cas, les techniques de réticulation ont été utilisées limitativement sur des pièces anatomiques ou des tissus contenant du collagène.

De façon plus exceptionnelle, elles ont été mises en oeuvre pour la réticulation d'articles préformés en collagène, essentiellement des films ou des feutres.

En tout état de cause, elles demeurent inopérantes dans le large domaine restant des applications à titre de biomatériaux.

Il a été proposé, par ailleurs, d'exploiter le pontage le plus couramment rencontré sur le plan biologique : la liaison disulfure -S-S-.

Il a ainsi été décrit dans l'article intitulé "Einbau von cystin-brücken in kollagen" - F. **SCHADE & H. ZAHN** - Angew, Chem 74, 904 (1962), la fixation directe, sans intermédiaire d'espacement, d'un dérivé de la cystine dans du collagène, pour tenter d'effectuer une réticulation par pontage S-S-.

Dans ce résumé succinct de leurs travaux, les auteurs prétendent avoir obtenu du collagène réticulé par des ponts disulfures.

L'agent réticulant mis en oeuvre est un dérivé de la cystine, dans lequel les deux fonctions amine de la cystine ont été bloquées par un groupement protecteur, du genre carbobenzoxyl.

Après greffage sur le collagène, les ponts disulfure ont été réduits puis réoxydés par l'oxygène de l'air (facteur d'autoréticulation) en milieu basique.

Cet article enseigne le greffage direct de cystine sur du collagène, sans utilisation d'un composé d'espacement.

Vingt ans après ce premier article, la demande de brevet européen n° **0 049 469** divulgue un pansement à base de collagène et de fibrinogène combinés. Dans ce document, on décrit l'introduction directe de fonctions thiols dans du collagène soluble extrait de tendons. Les fonctions thiols sont amenées par l'intermédiaire de la N-acétyl homocystéine thiolactone sans composé d'espacement la reliant au collagène.

La présente invention vise à proposer un collagène modifié, réticulable, soluble dans l'eau et/ou dans les solvants organiques polaires aprotiques, porteur de fonctions thiols libres ou substituées appartenant à des résidus de cystéine ou de ses analogues et apte à réticuler dans ces milieux, par formation de ponts disulfures, pour donner des gels ou des réticulats en présence d'oxydants doux, en permettant une excellente maîtrise de la cinétique et du taux de réticulation.

Un autre but de l'invention est de fournir un collagène "thiolisé", transformable en gels ou réticulats de densité de réticulation, donc de résistance mécanique, modulable à l'avance, de façon à être adaptable à toute application.

Un autre but de l'invention est de fournir un collagène, modifié, réticulable dont la souplesse et les performances de réticulation le consacrent comme une matière première particulièrement appropriée pour l'élaboration, par exemple par moulage ou extrusion, d'articles médicaux solides du type implants ou prothèses médicaux.

Aussi, c'est après avoir mené de nombreux essais et études que la demanderesse est parvenue à surmonter les obstacles auxquels s'était heurté l'art antérieur et à atteindre ces buts et d'autres en fixant des résidus cystéiques sur le collagène, au moins en partie par l'intermédiaire de composés d'espacement.

Ainsi, la présente invention concerne un nouveau collagène modifié, réticulable, soluble dans l'eau et/ou dans les solvants organiques polaires aprotiques, et contenant des fonctions thiols libres ou substituées, portées par des résidus de cystéine ou de ses dérivés, lesdits résidus étant fixés au collagène, au moins en partie, par l'intermédiaire de composés d'espacement.

De façon tout à fait avantageuse, le collagène modifié suivant l'invention est aisément façonnable et manipulable industriellement. Il permet d'obtenir des articles médicaux, du type implant, prothèse ou peau artificielle, atoxiques, non immunogènes et dont les propriétés mécaniques et biologiques sont parfaitement adaptées à l'application visée.

Au sens de la présente invention, le terme "réticulable" désigne indifféremment des collagènes modifiés aptes à s'autoréticuler spontanément en présence de l'oxygène de l'air, à température ambiante, éventuellement en présence d'agents auxiliaires doux, tels que des oxydants, n'intervenant pas directement dans la réaction et ne se retrouvant pas dans le produit réticulé.

L'excellente biocompatibilité de ce collagène modifié trouve, en partie, son origine dans le fait que les fonctions thiols libres ou substituées sont portées par des résidus de cystéine ou de ses analogues (ci-après indifféremment désignés sous le terme général : résidus "cystéiques"), à savoir par exemple la cystéine elle-même, la cystine, l'homocystéine, l'homocystine, la cystamine et la cystéamine.

Conformément à l'invention, les résidus "cystéiques" sont liés au collagène, au moins en partie par l'intermédiaire de composés d'espacement. Chaque composé d'espacement comprend, de préférence, plusieurs radicaux carboxyliques. Plus préférentiellement encore, le composé d'espacement est un motif hydrocarboné de nature carboxylique, de préférence issu d'un acide dicarboxylique apte à former un anhydride cyclique. L'acide dicarboxylique considéré peut être choisi dans la liste non limitative suivante : acide succinique, glutarique, phtalique, itaconique, maléique, l'acide succinique étant particulièrement préféré.

Ce composé d'espacement permet le greffage indirect d'au moins une partie des résidus "cystéiques" sur les acides aminés collagéniques à fonctions alcool ou amine libres. D'autres résidus "cystéiques" se fixent directement sur les acides aminés porteurs de groupements carboxyliques (acides glutamique et aspartique).

Le taux de substitution, par des fonctions thiols libres du collagène modifié suivant l'invention, peut varier dans une large plage de valeurs.

Ce collagène modifié passe aisément à l'état réticulé par oxydation des fonctions thiols et création de ponts disulfures dans un environnement oxydant doux. Dans des conditions physiologiques, in vivo, cela peut se produire par autooxydation par l'oxygène dissous ou par oxydation enzymatique, alors que dans des conditions non physiologiques, in vitro, l'oxydation peut s'effectuer à l'aide de réactifs non toxiques aux doses actives, tels que le peroxyde d'hydrogène ou de l'oxydène de l'air, en milieu faiblement basique e. g..

Le polymère réticulé peut être obtenu très stable et possédant de bonnes propriétés mécaniques.

L'invention concerne, également, à titre de produit nouveau, un collagène modifié, réticulable, soluble dans l'eau et/ou dans les solvants organiques polaires aprotiques, et contenant des fonctions thiols libres ou substituées portées par des résidus de cystéine ou de ses dérivés,
lesdits résidus :
- étant fixés au collagène, au moins en partie par l'intermédiaire de composés d'espacement,
- et répondant à la formule générale suivante : 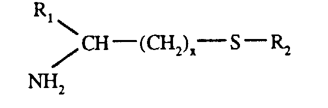
dans laquelle :
- R₁ = H, COOR'₁, avec R'₁ = chaîne carbonée aliphatique et/ou aromatique et/ou cyclique, de préférence alkylique, alkylénique, arylique ou aralkylénique et, plus préférentiellement encore, méthylique, éthylique ou allylique ;
- R₂ est l'hydrogène ou une chaîne carbonée éventuellement soufrée, aliphatique et/ou aromatique et/ou cyclique, de préférence choisie parmi les groupements de formule générale suivante : 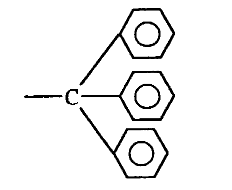 ou 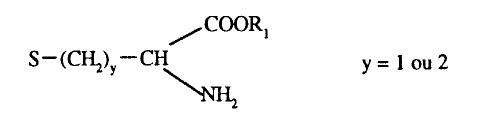
- x égal à 1 ou à 2.

Ces derniers peuvent être du même type que ceux décrits ci-dessus.

Ce collagène réticulé peut être obtenu à partir du collagène modifié, autoréticulable évoqué ci-dessus.

La présente invention concerne, par ailleurs, un procédé d'obtention d'un collagène modifié, réticulable, soluble dans l'eau et/ou dans les solvants organiques polaires aprotiques, et contenant des fonctions thiols libres.

Ce procédé consiste, essentiellement, à faire réagir le collagène de départ :
- voie I: soit avec le précurseur du composé d'espacement dans un premier temps, puis avec un résidu cystéique dans un second temps,
- voie II : soit avec une sous-unité réactionnelle, constituée par au moins le composé d'espacement lié à au moins un résidu cystéique.

Les étapes successives de la voie I sont les suivantes :
**a**_{**1**} solubilisation du collagène de départ dans au moins un solvant organique polaire aprotique,
- **b**_{**1**} - acylation et carboxylation du collagène solubilisé,
- **c**_{**1**} - activation des fonctions carboxyles libres du collagène,
- **d**_{**1**} - réaction du collagène activé avec un résidu cystéique à fonction(s) thiol(s) et, le cas échéant, à fonction(s) carboxylique(s) bloquée(s), de manière à obtenir un précurseur inerte du collagène modifié visé.

Suivant une première forme de mise en oeuvre de la voie I du procédé selon l'invention, il est prévu une étape supplémentaire **e**_{**1**} consistant en l'activation directe du précurseur inerte par formation de fonctions thiols libres ou substituées conduisant au collagène modifié réticulable visé. Cette activation peut notamment être réalisée par réduction.

Suivant une deuxième forme de mise en oeuvre de la voie I du procédé suivant l'invention, les étapes supplémentaires suivantes sont prévues :
- **e**_{**1**_{**1**}} - activation indirecte du précurseur inerte, de préférence par oxydation, conduisant à du collagène réticulé par l'intermédiaire de ponts disulfure intercaténaires,
- **f**_{**1**_{**1**}} - transformation, de préférence par réduction, du collagène réticulé en collagène modifié porteur de fonctions thiols libres ou substituées, stabilisées.

Les étapes **a**_{**1**} à **d**_{**1**}, **e**_{**1**}, **e**_{**1**_{**1**}} et **f**_{**1**_{**1**}} sont décrites en détail ci-après.

Conformément à la voie II, le procédé comprend, essentiellement, les étapes successives suivantes :
- **a**_{**2**} - solubilisation du collagène de départ dans au moins un solvant organique polaire,
- **b**_{**2**} - préparation de la sous-unité composé d'espacement-résidu cystéique à fonctions thiols protégées.
- **c**_{**2**} - activation des fonctions carboxyles libres de la sous-unité,
- **d**_{**2**} - mise en réaction du collagène et de la sous-unité activée, de manière à obtenir un précurseur inerte du collagène modifié visé.

Pour obtenir ce dernier, il suffit de mettre en oeuvre une étape supplémentaire **e**_{**2**} consistant en une réduction conduisant à du collagène SH.

Selon une alternative, l'étape **d**_{**2**} peut être suivie des étapes **e**_{**2**} et **f**_{**2**_{**1**}}, identiques aux étapes **e**_{**1**_{**1**}} et **f**_{**1**_{**1**}} décrites ci-dessus.

Les étapes **a**_{**2**} à **e**_{**2**}, **e**_{**2**_{**1**}} et **f**_{**2**_{**1**}} sont présentées plus en détail ci-après.

Le matériau de départ utilisé dans ce procédé peut être du collagène animal ou humain, de tous types et, de préférence, de type I et/ou III ou IV, solubilisable dans les solvants organiques polaires aprotiques, présentant ou non des télopeptides et employé, sous forme dénaturée (chaînes simples) ou non (triple hélice).

Il peut éventuellement s'agir d'un collagène modifié, par exemple, par acylation (e.g. succinylation) de ses fonctions aminées, ou bien par transformation en un sel d'acide, par exemple succinique.

Il va de soi que le matériau de départ peut être constitué par un ou plusieurs de ces différents types de collagène.

Conformément à l'étape **a**_{**1**} ou **a**_{**2**}, le collagène de départ est solubilisé dans un solvant organique polaire aprotique, tel que le diméthylsulfoxyde (DMSO), le diméthylacétamide (DMAC), le diméthylformamide (DMF) ou le N-méthylpyrrolidone (NMP).

Cette solubilisation est, de préférence, effectuée à l'aide d'un auxiliaire de solubilisation qui peut être un solvant, comme par exemple le méthanol ou un acide carboxylique correspondant, de préférence, à celui employé lors de l'étape **b**.

Des lors que le collagène de départ est un collagène avec télopeptides, il peut être soumis à un traitement préliminaire de réduction, alcalinisation, acidification et précipitation, approprié et connu en soi. Ce traitement vise à augmenter la solubilité dudit collagène dans le solvant de l'étape **b**_{**1**}.

L'étape **b**_{**1**} est une étape-d'acylation et de carboxylation du collagène solubilisé. Elle est, de préférence, effectuée à l'aide d'un anhydride de diacide carboxylique. Cette réaction permet la fixation par liaison covalente de l'une des extrémités COOH du diacide sur une fonction OH ou NH₂ libre des acides aminés. En final, la chaîne peptidique est porteuse de groupements COOH libres, constitués chacun par l'autre extrémité carboxylée du diacide.

La forme réactive devant se présenter sous forme d'anhydride, on choisit, en tant que diacide carboxylique, ceux qui sont aptes à former des anhydrides cycliques. Il s'agit, de préférence, de diacides comportant au moins 4 atomes de carbone.

A titre d'exemple, on peut citer la liste de composés suivants : acide succinique, glutarique, phtalique, itaconique, citraconique et maléique. Naturellement, il convient d'étendre cette liste aux dérivés de tous types des susdits acides.

Les anhydrides d'acide succinique ou glutarique sont particulièrement préférés.

Avantageusement, l'anhydride est mis à réagir avec le collagène en solution, en présence d'une base organique, de préférence du type tertiaire, telle que la triéthylamine, ou bien encore la N-méthyl ou N-éthyl morpholine.

Aprés extraction et lavage, on récupère un collagène substitué par le diacide considéré, à un taux modulable en fonction de la proportion d'anhydride mis en présence du collagène et/ou de la quantité de base mise en oeuvre.

D'une manière générale, compte tenu du nombre de fonctions réactives disponibles sur le collagène, le taux peut atteindre environ 22 acides aminés acylés pour cent acides aminés du collagène, de préférence 4 à 22. Ces collagènes acylés, par exemple succinylés, ayant un taux de substitution préférentiellement compris entre 4 et 22 radicaux acyles pour cent acides aminés, constituent de nouveaux produits intermédiaires stables.

Les collagènes substitués obtenus sont solubles dans l'eau à un pH supérieur à 5,5-7 et dans les solvants organiques polaires aprotiques, quel que soit l'anhydride utilisé.

A titre illustratif et non limitatif, on peut signaler que les collagènes substitués par de l'acide succinique sont solubles dans l'eau à un pH inférieur à 2,3 et supérieur à 5,5.

Après acylation/carboxylation, les collagènes présentent environ entre 10 et 30 % en nombre de résidus amino acides porteurs d'une fonction carboxylique. Il s'agit, en premier lieu, de ceux qui ont réagi avec l'anhydride acide, et, en second lieu, de l'acide glutamique et de l'acide aspartique présents dans le collagène de départ.

Selon la voie II, l'étape **b**_{**2**} consiste à préparer une sous-unité réactionnelle :
composé d'espacement-résidu cystéique
ou
composé d'espacement-résidu cystéique-composé d'espacement

Il s'agit, de préférence, d'un résidu cystéique du type de ceux décrits ci-dessus présentant, à chacune de ses extrémités, un reste d'acide dicarboxylique, tels que ceux cités ci-avant, par exemple une disuccinylcystamine.

L'étape **c**_{**1**} d'activation des fonctions carboxyles du collagène carboxylé et l'étape **c**_{**2**} d'activation de la sous-unité réactive sont, avantageusement, réalisées :
- soit à l'aide d'un halogénure d'acide carboxylique, de préférence un chlorure d'acide, conduisant à la formation d'anhydrides mixtes sur toute fonction carboxyle libre du collagène acylé ou de la sous-unité réactive,
- soit à l'aide de carbonyldiimidazole.

Dans le cas où un chlorure d'acide est utilisé, il est généralement choisi dans la famille des alkyl et/ou aryl-chloro-carbonates et des chlorures d'acides carboxyliques encombrés.

On retiendra préférentiellement l'éthyl chloroformate, mais également le chlorure de triméthyl acétyle.

L'activation s'opère dans un milieu solvant aprotique polaire (DMSO, DMF, DMAC, NMP, seul ou en mélange), de préférence en présence d'une base organique tertiaire, telle que la triéthylamine ou la N-méthyl ou N-éthyl morpholine, de préférence, la triéthylamine.

S'agissant du carbonyldiimidazole, l'activation s'opère dans un milieu solvant polaire aprotique (sans base organique).

Pour le collagène, cette réaction d'activation intéresse aussi bien les résidus carboxyliques introduits dans l'étape **b**_{**1**} que les carboxyles des acides aminés glutamique et aspartique, soit au total pour un collagène fortement acylé, environ 30 % en nombre des acides aminés.

Le collagène acylé, par exemple succinylé et activé, par exemple à l'aide d'éthylchloroformate, ainsi que la sous-unité activée constituent des intermédiaires réactionnels nouveaux.

L'étape **d**_{**1**} consiste à faire réagir le collagène activé avec un résidu "cystéique" à fonction(s) thiol(s) bloquée(s) et l'étape **d**_{**2**}**,** le collagène avec une sous-unité activée composé d'espacement-résidu cystéique, de manière à obtenir un précurseur inerte du collagène modifié visé.

Par résidus "cystéiques" au sens de la présente invention, on entend tout composé de formule générale : 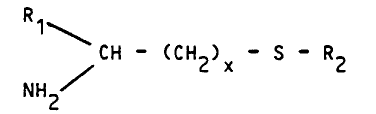 dans laquelle :
- **R**_{**1**} **= H, COOR'**_{**1**}**,** avec **R'**_{**1**} = chaîne carbonée aliphatique et/ou aromatique et/ou cyclique, de préférence alkylique, alkylénique, arylique ou aralkylénique et plus préférentiellement encore, méthylique, éthylique, ou allylique ;
- **R**_{**2**} est une chaîne carbonée éventuellement soufrée, aliphatique et/ou aromatique et/ou cyclique, de préférence choisie parmi les groupements de formule générale suivante : 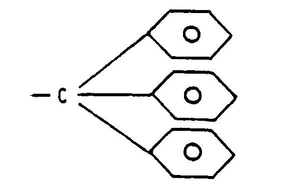 ou 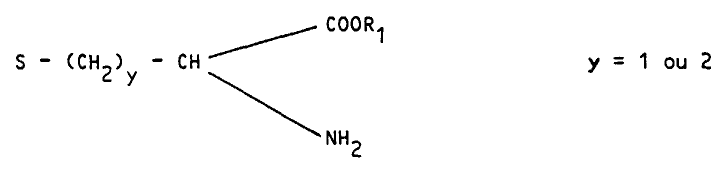
- **x** est égal à 1 ou à 2

Dans la voie I, le substituant en **R**_{**2**} constitue un moyen de protection des fonctions thiols de résidus cystéiques, afin d'éviter que celles-ci ne réagissent sur les COOH activés du collagène, lors de leur mise en contact.

Suivant une disposition préférée de la présente invention, on utilise, dans la voie I, à titre de résidu cystéique, la cystine diméthylester : 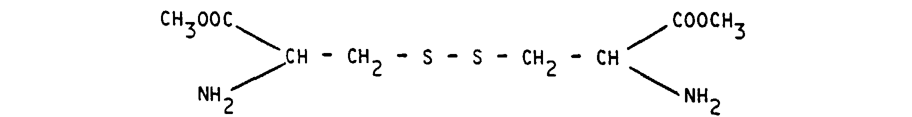 ou la S-triphényl méthyl cystéine méthylester :

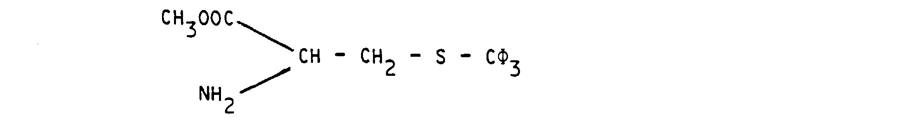

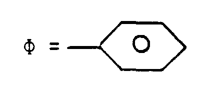

Le collagène substitué par le résidu cystéique, ou par la sous-unité composé d'espacement-résidu cystéique, constitue un précurseur inerte du collagène modifié que l'on cherche à obtenir. Il s'agit d'un intermédiaire réactionnel nouveau et stable.

Les étapes **e**_{**1**} et **e**_{**2**} du procédé conforme à l'invention consistent en une reconstitution des fonctions thiols, par élimination du groupement protecteur. Celle-ci peut être réalisée par réduction à l'aide d'un agent réducteur choisi, de préférence, parmi les mercaptans et/ou les sels réducteurs et/ou les composés réducteurs organiques.

Le mercaptan peut être le mercaptoéthanol, l'acide mercaptoacétique, la mercaptoéthylamine, le benzylmercaptan, le thiocrésol ou le dithiothréitol, ce dernier composé étant particulièrement préféré.

Comme sel réducteur, il est possible de retenir, par exemple, le borohydrure de sodium ou l'hydrogénosulfite de sodium.

Les phosphines, telles que la tributylphosphine conviennent bien à titre de composé réducteur organique.

On peut utiliser des mélanges d'agent réducteur, comme par exemple une combinaison de mercaptoéthanol/borohydrure de sodium.

La réduction peut être réalisée en milieu aqueux basique ou neutre, dans des solvants organiques ou dans des mélanges de solvants organiques en présence ou en absence d'eau.

Ces étapes **e**_{**1**} et **e**_{**2**} de réduction sont mises en oeuvre, notamment, lorsque le groupement **R**_{**2**} du résidu cystéique considéré est du type :

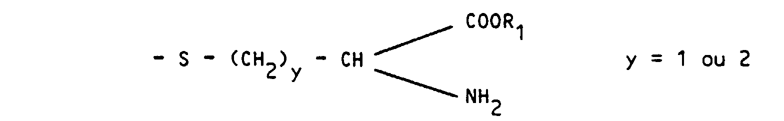

Les étapes **e**_{**1**_{**1**}} et **e**_{**2**_{**1**}} consistent en une oxydation du précurseur inerte, à l'aide d'un agent oxydant, tel que l'iode en solution alcoolique, par exemple méthanolique, dans un milieu réactionnel à base de solvant polaire organique aprotique, caractéristique du procédé suivant l'invention, tel que le DMF. On obtient du collagène réticulé par des ponts disulfure.

Pour obtenir le collagène réticulable à fonctions SH libres ou substituées, il convient de mettre en oeuvre l'étape **f**_{**1**_{**1**}} ou **f**_{**2**_{**1**}} de réduction de ce collagène réticulé.

Cette réduction est du type de celle mise en oeuvre dans le cadre de l'étape **e**_{**1**} ou **e**_{**2**} décrite ci-dessus.

Les étapes **e**_{**1**} et **f**_{**1**} sont mises en oeuvre lorsque le précurseur inerte présente un résidu cystéique ayant un substituant **R**_{**2**} du type CΦ₃.

Sous un autre aspect, l'invention concerne, également, un procédé d'obtention d'un collagène réticulé, insoluble dans l'eau et/ou dans les solvants organiques polaires aprotiques, consistant à mettre en oeuvre les étapes **a**, **b**, **c**, **d**, et **e**_{**1**_{**1**}} ou **e**_{**2**_{**1**}} du procédé décrit ci-dessus.

Les résidus cystéiques SH greffés sur les chaînes de collagène sont oxydés pour donner des ponts disulfure entre ces molécules. Cette réaction conduit à la formation d'un réseau tridimensionnel, insoluble dans les milieux physiologiques et soluble dans des milieux réducteurs capables de réduire les ponts disulfure.

Le nombre de liaisons formées entre ces différentes molécules dépend du degré de substitution et des conditions d'oxydation.

S'agissant du taux de réticulation, il est déterminant au regard de la résistance mécanique et de la cinétique de la biodégradabilité des réticulats obtenus.

Le collagène réticulable et le collagène réticulé suivant l'invention ne contiennent que des molécules ou dérivés de nature biologique, susceptibles d'être facilement métabolisés pour donner des composés reconnus comme métabolites par les organismes animaux.

Les réactifs utilisés lors des modifications chimiques sont, soit transformables en produits non toxiques, soit facilement éliminables par des procédés non dénaturants, comme la dialyse par exemple.

Le collagène modifié sous forme réduite ne contient pas de fonctions activées résiduelles et le collagène réticulé oxydé ne peut contenir que des fonctions thiols n'ayant pas réagi. Ces fonctions ne sont pas toxiques, puisque naturellement présentes dans un grand nombre de protéines animales.

Les procédés d'oxydation ne font pas appel à des substances toxiques, ni à des conditions agressives pour les tissus vivants.

L'invention offre la possibilite très appréciable de pouvoir contrôler l'ensemble des phénomènes de réticulation du collagène, dont notamment la cinétique et le taux. Ceci est particulièrement utile pour la réalisation d'objets moulés ou extrudés, du type implants, prothèses, lentilles épicornéennes, etc.

Un autre avantage non négligeable de l'invention est qu'elle permet de moduler les propriétés mécaniques, en contrôlant le nombre de résidus cystéiques introduits par unité de masse du collagène. Il est également possible de cibler précisément une classe de produits réticulés conformes au cahier des charges, établi en fonction de l'application visée et définissant les contraintes mécaniques et les cinétiques de biodégradation souhaitées.

Les produits et les procédés suivant l'invention trouvent des applications immédiates, d'une part, en médecine humaine ou vétérinaire et, d'autre part, dans le domaine de la biologie.

En médecine humaine ou vétérinaire, il peut s'agir d'implants, par exemple ophtalmologiques, de prothèses, par exemple osseuses, de pansements sous forme de films ou de feutres, de tissus artificiels (épiderme, vaisseaux, ligaments, os), de systèmes, soit d'encapsulation (microsphères, microcapsules) permettant la libération contrôlée de principes actifs in vivo, soit de bioencapsulation, de revêtements de biocompatibilisation d'articles médicaux implantables ou bien encore de fils de suture. Ces produits peuvent, également, être associés à des charges minérales (hydroxyapatite, poudre de corail, ...) en vue d'être utilisés en tant qu'agents de comblement osseux ou cartilagineux.

En biologie, les matériaux suivant l'invention constituent d'excellents supports pour cultures cellulaires bidimensionnelles (films) et tridimensionnelles (feutres).

Le collagène réticulé selon l'invention peut être utilisé seul ou en mélange avec des polymères biologiques modifiés ou non ou des polymères synthétiques.

D'autres applications intéressantes des produits selon l'invention se situent dans le domaine de l'adhésion, e. g. : articles biomatériaux et/ou adhésifs, colles biologiques ou chirurgicales.

D'autres avantages, variantes et possibilités d'application de la présente invention ressortent bien des exemples ci-après.

### EXEMPLES

### EXEMPLE 1 : Synthèse de collagène-succinyl-cystéinyle sous forme reticulée et sous forme réticulable à partir d'atélocollagène (étapes a₁ à d₁, e_{1₁} et f_{1₁}) (voie I)

### Etape a₁: Solubilisation de l'atélocollagène de départ :

32 g d'acide succinique sont dissous dans 270 ml de méthanol. 25 g d'atélocollagène (origine bovine - type I + III - provenance Société **SAPUC**) sont mis en suspension dans cette solution. Après gonflement pendant 1 h, 290 ml de DMSO ou 375 ml de NMP sont ajoutés et le milieu est agité jusqu'à dissolution complète à 20° C. Le milieu est alors filtré sur filtre 45 µm et le méthanol est évaporé sous pression réduite (environ 0,3 mbar).

### Etape b₁ : Activation et carboxylation du collagène solubilisé :

A la solution collagénique exempte de méthanol obtenue à l'issue de l'étape **a**_{**1**}, sont ajoutés 30 g (300 mmoles) d'anhydride succinique, puis après dissolution complète 75 ml (538 mmoles) de triéthylamine fraîchement distillée, au goutte à goutte pendant 10 min. La température est maintenue à 25° C par un système externe de thermostatisation. La solution obtenue est agitée pendant 3 à 15 heures, puis le collagène succinylé est précipité par addition de deux volumes d'acétate d'éthyle. Le précipité est ensuite lavé par deux bains successifs de 250 ml d'acétone. Le résidu solide obtenu est dissous dans 300 ml d'eau distillée mise à pH 6,5 puis la solution est dialysée contre de l'eau distillée amenée à pH 2 (acidification par HCl 6N). Après lyophilisation, 21,5 g de collagène succinylé sur 20 % des acides aminés sont obtenus.

Le taux de succinylation est déterminé par deux techniques :
- dosage enzymatique de l'acide succinique après hydrolyse du collagène en milieu acide,
- dosage potentiométrique des fonctions carboxyliques.

### Etapes c₁ et d₁: Activation d'atélocollagène succinylé et greffage de la S-Triphénylméthyl cystéine éthyl ester :

4 g de collagène succinylé à 20 % (10,54 mmol acidité totale) obtenus lors de l'étape précédente sont mis en solution dans 80 ml de DMF anhydre. Après totale dissolution, 1,72 ml de triéthylamine (12 mmol) sont ajoutés sous forte agitation. Le milieu réactionnel est mis à - 5° C avant addition au goutte à goutte de 1,92 ml d'éthylchloroformate (20 mmol). Après 15 min, le collagène succinylé est activé et l'étape **c**_{**1**} prend fin.
L'étape **d**_{**1**} est amorcée par l'addition, dans le milieu réactionnel, de 7,2 g de S-triphénylméthyl cystéine éthyl ester (18 mmol), obtenu par mise en présence de cystéine éthyl ester avec du triphénylméthanol et du BF₃ éthérate (température supérieure à 50° C). Le milieu réactionnel est alors laissé 16 h sous agitation à 20-25° C. Le dérivé collagénique formé est ensuite précipité par addition de 400 ml d'acétate d'éthyle. Après redissolution dans le DMF et reprécipitation à l'acétate d'éthyle, le dérivé collagénique obtenu est lavé par du méthanol, puis séché sous pression réduite à 20-25° C. 4,5 g de produit sec sont ainsi obtenus.

### Etapes e_{1₁} et f_{1₁} : Déblocage du groupement protecteur - "CΦ₃"-obtention du collagène réticulé par des ponts disulfure :

Les 4,5 g du produit formé à l'étape **d**_{**1**} sont laissés 3 h sous agitation dans 45 ml de DMF. A ce stade, le dérivé est entièrement solubilisé. 1,45 g (7 mmol) d'iode solubilisés dans 20 ml de méthanol sont ajoutés.
Il y a formation d'un gel. Le milieu est laissé 18 h, puis lavé plusieurs fois avec un mélange eau:acétone/50:50 (v:v) jusqu'à décoloration complète. Après trois lavages à l'acétate d'éthyle, le produit est séché. 3,46 g de dérivé final sec sont obtenus.
Le degré de substitution du collagène obtenu est de l'ordre de 3 à 5 résidus cystéine pour 100 acides aminés par dosage des groupements thiol au DTNB.
Ce dérivé réticulé peut être réduit par dithiothréitol pour donner le dérivé collagène thiol aisément réticulable à l'air, à l'eau oxygénée ou à l'iode.

### EXEMPLE 2 : Synthèse de collagène-succinyl-cystéinyle sous forme réticulée et sous forme réticulable à partir d'un sel succinyle d'atélocollagène (voie I)

### Etape préliminaire : Obtention du sel succinyle d'atélocollagène :

12 g d'acide succinique (102 mmol) sont dissous dans 225 ml d'isopropanol. 12 g d'atélocollagène sont ajoutés dans ce milieu réactionnel que l'on met sous agitation magnétique pendant 2 h à 20-25° C.
Après filtration sur tamis 50 µm, les fibres de collagène sont essorées puis lavées deux fois avec 150 ml d'isopropanol avant d'être à nouveau tamisées.
13 g de sel succinylé d'atélocollagène sec sont alors récupérés après 4 h de séchage sous pression réduite à 25-30° C.

### Etapes a₁ et b₁ : Solubilisation du sel et synthèse d'atélocollagène succinylé à partir de son sel succinylé :

10 g de sel succinylé d'atélocollagène sont dissous dans 240 ml d'un mélange DMF:DMSO/60:40 (v:v) sous agitation magnétique. 3,9 g d'anhydride succinique (39 mmol) sont ajoutés au milieu ainsi que 8,7 ml de triéthylamine fraîchement distillée (62,5 mmol). Le milieu réactionnel est laissé 24 h sous agitation magnétique à 20-25° C, puis dialysé contre de l'eau distillée. Après acidification à pH 2-4, le produit est précipité à l'acétone avant d'être dialysé contre de l'eau à pH 2. Après lyophilisation, 8 g de dérivé sec succinylé à 9,5 % (détermination par potentiométrie) sont obtenus.

### Etapes c₁ et d₁ : Activation d'atélocollagène succinylé et greffage de la S-triphénylméthyl cystéine éthyl ester :

4,5 g d'atélocollagène succinylé à 9,5 % (acidité totale 8,37 mmol) sont dissous dans 90 ml de DMF anhydre pendant 18 h sous agitation magnétique à 20-25° C. Après avoir refroidi le milieu à 5/- 10° C, 1,575 ml de triéthylamine (11,3 mmol) sont ajoutés avant addition au goutte à goutte de 1,2 ml d'éthylchloroformate (12,6 mmol).
Après 15 min d'agitation à - 5/-10° C, le collagène succinylé est activé et l'étape **c**_{**1**} prend fin.
L'étape **d**_{**1**} est amorcée par l'addition de 6,8 g (16,5 mmol) de S-triphénylméthyl cystéine méthyl ester dans le milieu réactionnel qui est laissé 1 h sous agitation magnétique à - 5° C, puis 16 h à 20-25° C.
Le dérivé formé est ensuite précipité par 400 ml d'acétate d'éthyle. Après redissolution dans le DMSO et reprécipitation dans l'acétate d'éthyle, le dérivé est lavé par du méthanol puis séché sous pression réduite pour donner 7,6 g de produit sec.

### Etapes e_{1₁} et f_{1₁} : Déblocage du groupement protecteur - "CΦ₃"-obtention du collagène réticulé par des ponts disulfure ::

Les 7,6 g du produit formé à l'étape **d**_{**1**} sont laissés 3 h sous agitation dans 80 ml de DMF. 100 ml de méthanol sont rajoutés. A ce stade, le dérivé est totalement solubilisé. 1,77 g (7 mmol) d'iode solubilisé dans 20 ml de méthanol sont alors ajoutés.
Il y a formation d'un gel. Le milieu est laissé 18 h, puis lavé plusieurs fois avec un mélange eau:acétone/50:50 (v:v) jusqu'à décoloration complète. Après trois lavages à l'acétate d'éthyle, le produit est séché pour donner 6 g de dérivé final sec.
Le degré de substitution du collagène modifié obtenu est estimé à 11 résidus cystéine pour 100 acides aminés par dosage des groupements thiol au DTNB.
Ce dérivé réticulé peut être réduit au dithiothréitol pour donner le dérivé collagène thiol aisément réticulable à l'air, à l'eau oxygénée ou à l'iode.

### EXEMPLE 3 : Synthèse de collagène-succinyl-cystéinyle (réticulé et réticulable) à partir de collagène avec télopeptides bovin de type I + III (voie I)

### Etapes a₁ et b₁ :

2,5 g de collagène bovin avec télopeptides type I + III sont dissous à 50° C dans 80 ml d'eau et la solution fluide obtenue est agitée à cette température pendant 1 h. 0,25 g de dithiothréitol (1,11 mmol) sont alors ajoutés après refroidissement de la solution de collagène jusqu'à environ 30° C et le pH est amené à 9,5 par de l'hydroxyde de sodium. La solution obtenue est agitée pendant 1 h, puis ramenée à pH 2,5-3 par de l'acide chlorhydrique. Le collagène est alors précipité par deux volumes d'acétone, lavé dans ce même solvant, puis récolté par filtration et séché sous pression réduite.
Le collagène en poudre obtenu précédemment (2,2 g) est mis en suspension dans 20 ml de méthanol et agité pendant 15 min, puis 40 ml de diméthyl-sulfoxide anhydre contenant 0,4 g d'acide succinique (3,39 mmol) sont ajoutés. Après 30 min d'agitation le méthanol est évaporé sous pression réduite à 30° C. A la solution obtenue sont ajoutés 2,91 g (29,1 mmol) d'anhydride succinique puis, après dissolution de ce dernier, 4,5 ml (32,3 mmol) de triéthylamine fraîchement distillée. Le milieu est agité pendant 2 h à 20° C. Le collagène succinylé est précipité par 200 ml d'acétone, puis récolté par filtration. Le précipité est alors mis en dissolution dans 80 ml d'eau à pH 8, la solution est centrifugée 20 min à 15 000 g et le surnageant est dialysé contre de l'eau distillée maintenue à pH voisin de 2 par de l'acide chlorhydrique. Le dialysat est lyophilisé pour donner 2,3 g de collagène succinylé dont le taux de substitution, déterminé par potentiométrie, est de 18 %.

### Etapes c₁, d₁ et e₁ :

1 g de collagène bovin succinylé à 18 % (environ 2,52 mmol de fonction carboxyle) est solubilisé dans 25 ml de DMSO anhydre. A cette solution très visqueuse sont ajoutés 0,55 g (3,39 mmol) de carbonyldiimidazole en poudre. Le milieu est dégazé sous pression réduite et agité pendant 45 min. L'addition de 0,8 g (2,35 mmol) de cystine diméthyl ester en solution dans 5 ml de DMSO anhydre donne, en quelques minutes, un gel qui est laissé au repos pendant 18 h à l'obscurité. Le gel est dispersé dans 100 ml d'acétone et agité pendant 2 h, puis il est lavé dans plusieurs bains d'acétone, récolté par filtration et séché sous pression réduite.
Le collagène modifié obtenu (1 g) est mis en suspension dans 50 ml d'eau maintenue à pH 9,5. 0,4 g (2,6 mmol) de dithiothréitol en solution dans 10 ml d'eau à pH 9,5 sont ajoutés et le milieu est agité pendant 18 h à 20-25° C. La solution obtenue est centrifugée pendant 15 min à 15 000 g, le surnageant est acidifié jusqu'à pH 1,8 et le gel obtenu est dialysé contre de l'eau distillée à pH 1,5-1,8. Le dialysat est lyophilisé pour donner 0,985 g de collagène modifié avec un taux de substitution d'environ 9 fonctions thiol pour 100 acides aminés du collagène de départ.

### EXEMPLE 4 : Synthèse de collagène-succinyl-cystéamine à partir de collagène avec télopeptides bovin de type I + III (voie I)

Le protocole est identique à celui de l'exemple 3. 1,05 g de collagène thiolisé sont obtenus à partir de 1 g de collagène succinylé. Le taux de greffage est estimé à 14 % par rapport aux acides aminés du collagène de départ.

### EXEMPLE 5 : Synthèse de collagène-succinyl-cystéaminyle à partir du collagène avec télopeptides et d'une sous-unité réactionnelle disuccinyl-cystamine (voie II)

2,2 g de collagène acidosoluble (environ 0,78 mmol de résidus lysyl) sont agités pendant 15 min dans 75 ml de méthanol, puis sont ajoutés 125 ml de diméthylsulfoxyde. Le mélange est agité à 40° C pendant 15 min, puis le méthanol est évaporé sous pression réduite. 0,707 g (1,564 mmol) du diimidazolide de la disuccinyl cystamine (obtenue par réaction en milieu aqueux basique de l'anhydride succinique et de la cystamine) sont dissous dans 20 ml de DMSO et la solution obtenue est ajoutée à la solution du collagène. Le mélange est agité à 20-25° C pendant 20 h, puis le gel obtenu est dispersé dans 200 ml d'acétone, lavé par 2 x 100 ml de ce solvant, puis lavé en 2 h par 2 x 500 ml d'eau maintenue à pH 7-9,5 par de l'hydroxyde de sodium.
Le gel est récolté par filtration, puis placé dans 50 ml d'une solution aqueuse de dithiothreitol (0,5 g, 3,25 mmol) ajustée à pH 9,5 par de l'hydroxyde de sodium. Après 18 h d'agitation à 20-25° C, le milieu est porté à 40° C pendant 15 min, puis centrifugé à 15 000 g pendant 10 min. Le surnageant est alors acidifié à pH 1,8 et dialysé contre de l'eau à ce même pH. Le milieu collagénique est alors lyophilisé pour donner 1,8 g de collagène thiolisé sur 3 % des acides aminés du collagène de départ.

### EXEMPLE 6 : Synthèse de collagène-succinyl-cystéaminyle à partir d'atélo-collagène bovin de type I + III (voie I)

10 g d'atélo-collagène succinylé à 18 % (environ 25 mmol de fonctions carboxyliques) sont dissous dans 150 ml de diméthyl-sulfoxide anhydre. La solution visqueuse obtenue est dégazée sous pression réduite, puis 9,1 g (56 mmol) de carbonyldiimidazole en poudre lui sont ajoutés, en une seule fois, à une température de 20-25° C. Après dissolution de ce dernier, la solution obtenue est dégazée sous pression réduite pendant 1 à 2 h. 7,6 g (33,77 mmol) de chlorhydrate de cystamine sont dissous à 40° C dans 50 ml de DMSO anhydre, et la solution obtenue est ajoutée en une seule fois sous forte agitation à la solution de collagène activé. Le milieu gélifié obtenu en quelques minutes est laissé au repos pendant 3-24 h à 20-25° C. Le gel est alors dispersé dans 400 ml d'acétate d'éthyle, récupéré par filtration, puis lavé par deux fois dans 300 ml d'acétone. Le produit granuleux obtenu est alors dispersé dans de l'eau maintenue à pH 10 par de l'hydroxyde de sodium pendant 16 h, puis récolté par filtration. Le produit obtenu est mis en suspension dans 90 ml d'eau à pH 10, puis 3,85 g (25 mmol) de dithiothréitol en solution dans 20 ml d'eau à pH 10 sont ajoutés. Le mélange obtenu est agité pendant 16 h à 20° C et centrifugé à 15 000 g pendant 15 min.
Le surnageant est acidifié jusqu'à pH 1,8 par de l'acide chlorhydrique, puis dialysé contre de l'eau à pH 1,5-2. Le dialysat est lyophilisé pour donner 10,7 g de collagène thiolisé. Le dosage des fonctions thiol par la méthode du DTNB indique un taux de substitution de 15 % par rapport aux acides aminés du collagène, soit un taux d'environ 55 % par rapport aux fonctions carboxyliques du collagène succinylé de départ.

### EXEMPLE 7 : Synthèse de collagène-succinyl-cystéinylé à partir d'atélo-collagène bovin de type I + III (voie I)

10 g d'atélo-collagène succinylé à 18 % sont activés dans les mêmes conditions que dans l'exemple 6. 8,5 g (25 mmol) de cystine diméthyl ester sont dissous dans 10 ml de DMSO anhydre, puis la solution obtenue est ajoutée à la solution de collagène activé. Le milieu est agité pendant 16-60 h à 20-25° C. Le gel obtenu est dispersé sous forte agitation dans trois volumes d'acétone, lavé par trois fois dans 150 ml de ce solvant, puis récolté par filtration. Le collagène modifié peut alors être obtenu sous forme de poudre par évaporation sous pression réduite de l'acétone résiduelle ou utilisé directement dans l'étape suivante.
Le collagène modifié est mis en suspension dans 100 ml d'eau maintenue à pH 9,5 par de l'hydroxyde de sodium. A cette suspension est ajouté 3,85 g (25 mmol) de dithiothréitol en solution dans 20 ml d'eau à pH 9,5. Le mélange est agité pendant 16-24 h à 20-25° C et centrifugé à 15 000 g pendant 15 min. Le surnageant est acidifié à pH 1,8 par de l'acide chlorhydrique, puis dialysé contre de l'eau à pH 1,5-2, jusqu'à obtenir un test négatif au DTNB dans les eaux de dialyse. Le dialysat est lyophilisé. Le dosage des fonctions thiol par la méthode au DTNB indique un taux de substitution d'environ 9 % par rapport aux acides aminés du collagène.

### EXEMPLE 8 : Synthèse de collagène-succinyl-cystéaminyle à partir d'atélocollagène (voie II)

2,4 g d'atélo-collagène bovin, type I + III (environ 0,855 mmol de résidus lysyl) sont dissous dans 30 ml de DMSO anhydre par la méthode décrite dans l'exemple 3 étape **b**_{**1**}.
0,6 g (1,7 mmol) de disuccinyl-cystamine sont dissous dans 20 ml de diméthylformamide auxquels sont ajoutés 0,55 g (3,41 mmol) de carbonyldiimidazole en poudre. La solution est dégazée sous pression réduite et agitée pendant 2 h.
Le milieu hétérogène obtenu est ajouté à la solution de collagène obtenue précédemment et la solution obtenue est agitée à 20-25° C pendant 18 h. Le collagène est alors précipité par addition de trois volumes d'acétone et collecté par filtration. Le précipité est alors dispersé dans 200 ml d'eau maintenue à pH acide et la suspension est agitée pendant 18 h.
Le précipité lavé est dispersé dans 50 ml d'eau maintenue à pH 10 par de l'hydroxyde de sodium. A la suspension obtenue sont ajoutés 0,26 g (1,7 mmol) de dithiothréitol en solution dans 5 ml d'eau à pH 10. Le milieu est agité pendant 24-48 h à 20-25° C, acidifié par de l'acide chlorhydrique jusqu'à pH 1,8 et le gel obtenu est dialysé contre de l'eau à pH 1,5-2 puis lyophilisé pour donner 2,3 g de collagène modifié. Le dosage des fonctions thiol par la méthode au DTNB indique un taux de substitution de 3,1 % par rapport aux acides aminés du collagène de départ correspondant à un taux de substitution d'environ 100 % des résidus lysyl.

### EXEMPLE 9 : Synthèse de collagène-succinyl cystéinyle à partir d'atélo-collagène (voie I)

4 g d'atélo-collagène (environ 1,42 mmol de résidus lysyl) sont dissous dans du DMSO selon le protocole décrit dans l'exemple 2. 0,7 g (3 mmol de fonction carboxylique) de disuccinyl cystine diméthyl ester sont dissous dans 10 ml de DMSO, puis 0,486 g (3 mmol) de carbonyldiimidazole sont ajoutés. La solution est agitée et dégazée sous pression réduite pendant 1 h, puis elle est ajoutée à la solution de collagène. Après 72 h de repos à 20-25° C, le milieu réactionnel est dispersé dans 300 ml d'acétone, la phase solide est lavée pendant 1 h dans 200 ml d'acétone, puis 2 x 1 h dans 500 ml d'eau maintenue à pH 9-9,5 par de l'hydroxyde de sodium. Le gel est récolté par filtration, puis il est dispersé dans 70 ml d'eau à pH 9,5.
A cette suspension est ajoutée une solution aqueuse à pH 9,5 de dithiothréitol (6 ml, 0,7 g, 4,54 mmol). Après 18 h de réaction, le milieu est chauffé 10 min à 30° C, puis centrifugé 15 min à 15 000 g. Le surnageant est traité comme dans l'exemple 7 pour donner 3,9 g de collagène thiolisé sur 2,4-2,8 % des acides aminés du collagène de départ.

### OXYDATION-RETICULATION DES COLLAGENES THIOLISES

Tous les collagènes thiolisés sont oxydables et réticulables en présence d'oxydants. Suivant les conditions d'oxydation des collagènes en solution (solution aqueuse, organique ou mixte, température, force ionique, pH, concentration) et la nature de l'oxydant, la réticulation se traduit par la formation d'un gel, d'un précipité, par variation de la viscosité de la solution. Parmi les oxydants utilisés, on peut citer l'oxygène seul, l'oxygène en présence de rayonnement ultraviolet, le peroxyde d'hydrogène en milieu acide neutre ou basique, l'iode en solution alcoolique ou aqueuse, la réticulation peut également être réalisée sur du collagène thiolisé non solubilisé, sous forme de films ou de poudres, par exemple par immersion dans des solutions oxydantes.
Tous les collagènes oxydés sont insolubles en milieu aqueux et organique, mais peuvent être solubilisés dans des solutions réductrices à pH basique comme, par exemple, une solution aqueuse de dithiothréitol à pH 9,5.

### EXEMPLE 10 : Formation d'un gel

0,5 g (contenant environ 0,16 mmol de fonction thiol) d'atélocollagène issu de l'exemple 8, sont dissous à 40° C dans 10 ml d'eau, puis la solution est amenée à pH 7 par de l'hydroxyde de sodium et à pH 8,2 par du tampon carbonate 1M à pH 9. La solution est filtrée à 0,22 micromètres puis gélifiée par abaissement de la température.
Le gel thermoréversible obtenu est plongé dans 150 ml de tampon borate de sodium 0,15 M à pH 8,2 contenant 0,15 % de peroxyde d'hydrogène et maintenu dans cette solution sous faible agitation pendant 6 à 24 h. Le gel transparent obtenu est alors lavé dans plusieurs bains d'eau et peut être conservé dans une solution aqueuse d'éthanol à 25 %.
Ce gel n'est plus thermoréversible et ne contient plus de fonction thiol libre.

### EXEMPLE 11 : Formation d'un film

0,7 g de collagène issu de l'exemple 8, (contenant environ 0,23 mmol de fonction thiol) sont dissous à 40° C dans 50 ml d'eau, puis la solution est amenée à pH 8,2, comme dans l'exemple 10. Après filtration à 0,22 micromètres, la solution est coulée sur boîte en polystyrène 120 x 120 mm et évaporée à l'air libre à 20-25° C. Le film obtenu est alors plongé dans 100 ml d'une solution de peroxyde d'hydrogène (exemple 10) pendant 1 à 5 h, puis lavé par de l'eau. Ce film peut être conservé à l'état sec ou dans une solution aqueuse d'éthanol à 25 %.

### EXEMPLE 12 : Evaluation ex vivo de l'adhésion tissulaire

L'évaluation des propriétés adhésives des produits selon l'invention a été réalisée sur des tissus musculaires de lapin (râble). Ces tissus sont conservés à 4° C dans du sérum physiologique pendant 48 heures au maximum. Le tissu de lapin est découpé dans le sens des fibres à l'aide d'une trancheuse électrique (épaisseur des tranches : 2,5 + 0,5 mm), puis des carrés de 25 mm x 25 mm sont découpés dans les tranches obtenues. Les essais sont réalisés sur un appareil de traction usuel, par exemple de type **Adamel Lhomarcy** de type DY 34 muni d'un capteur de force de 100 N. Cet appareil permet d'obtenir les courbes force-déplacement. Il est possible d'en déduire la force maximale d'arrachement ou force d'adhésion (Fₘₐₓ) et l'énergie adhésive mise en jeu peut être calculée d'après l'aire sous la courbe.
Dans chaque type d'essai, deux éprouvettes de tissu de lapin sont fixées à l'aide d'une colle cyanoacrylique (par exemple vendue sous la marque **LOCTITE**® superglue, liquide ou gel) sur des supports inertes, verres ou cartons très rigides de dimension supérieure.
Les tests sont réalisés après un contact de 3 min sous une pression de 4 N.
Une solution à 5 % de collagène succinylé-cystéamine (préparée selon l'exemple 4), est réalisée sous atmosphère inerte. Son pH est ajusté entre 7 et 8. Avant le test, les surfaces des tissus sont imbibées superficiellement d'une solution de **BETADINE**® dermique (polyvinyl pyrolidone iodée).
100 µl de solution de collagène sont alors rajoutés entre les deux tissus et on observe une gélification progressive au contact de la solution iodée (oxydant).
Après 3 min de collage sous pression (4 N), on obtient une force adhésive F = 3,5 ± 1,9 N (moyenne de 14 essais) et une énergie d'adhésion E = 7,0 ± 3,1 mJ.
Ces valeurs sont intéressantes et peuvent être comparées aux valeurs obtenues dans le même test pour les colles commerciales à base de fibrine. Ainsi, pour la colle **BIOCOL**^{R} (dépôt de 200 ml) les valeurs d'adhésion obtenues sont : F = 2,5 ± 1,6 N et E = 3,8 ± 2,0 mJ (moyenne de 17 essais).

## Revendications

1. Collagène modifié, réticulable, soluble dans l'eau et/ou dans les solvants organiques polaires aprotiques, et contenant des fonctions thiols libres ou substituées portées par des résidus cystéiques,
lesdits résidus :
- étant fixés au collagène, au moins en partie par l'intermédiaire de composés d'espacement,
- et répondant à la formule générale suivante : 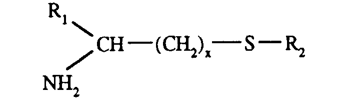
dans laquelle :
- R₁ = H, COOR'₁, avec R'₁ = chaîne carbonée aliphatique et/ou aromatique et/ou cyclique ;
- R₂ est l'hydrogène ou une chaîne carbonée éventuellement soufrée, aliphatique et/ou aromatique et/ou cyclique.

2. Collagène modifié selon la revendication 1, caractérisé en ce que le composé d'espacement est un motif hydrocarboné à fonction carboxylique.

3. Collagène modifié selon la revendication 2, caractérisé en ce que le motif hydrocarboné à fonction carboxylique est issu d'un acide dicarboxylique apte à former un anhydride cyclique.

4. Collagène modifié selon la revendication 2 ou 3, caractérisé en ce que l'acide dicarboxylique considéré est choisi dans le groupe de composés suivants : acide succinique, glutarique, phtalique, itaconique, citraconique et maléique, ainsi que leurs dérivés.

5. Collagène modifié selon la revendication 1, caractérisé en ce que les résidus porteurs des fonctions thiols libres ou substituées sont choisis parmi le groupe de composés suivants : cystéine, cystine, homocystéine, homocystine, cystéamine, cystamine.

6. Collagène réticulé, insoluble, caractérisé en ce que ses pontages intercaténaires sont constitués, au moins partiellement, par des ponts disulfure formés par des résidus cystéiques comme défini dans la revendication 1 fixés au collagène, au moins en partie par l'intermédiaire de composés d'espacement.

7. Collagène réticulé selon la revendication 5, caractérisé en ce qu'il est obtenu à partir du collagène selon l'une quelconque des revendications 1 à 5.

8. Procédé d'obtention d'un collagène modifié, réticulable ou autoréticulable, soluble dans l'eau et/ou dans les solvants organiques polaires aprotiques, et contenant des fonctions thiols libres ou substituées, caractérisé en ce qu'il comprend, essentiellement, les étapes successives suivantes :
**a**_{**1**} solubilisation du collagène de départ dans au moins un solvant organique polaire aprotique,
**b**_{**1**} acylation et carboxylation du collagène solubilisé,
**c**_{**1**} activation des fonctions carboxyles libres du collagène,
**d**_{**1**} réaction du collagène activé avec un résidu cystéique comme défini dans la revendication 1 à fonction(s) thiol(s) et, le cas échéant, à fonction(s) carboxylique(s) bloquée(s), de manière à obtenir un précurseur inerte du collagène modifié visé,
**e**_{**1**} activation directe du précurseur inerte conduisant au collagène modifié porteur de fonctions thiols libres ou substituées, stabilisées.

9. Procédé d'obtention d'un collagène modifié, réticulable ou autoréticulable, soluble dans l'eau et/ou dans les solvants organiques polaires aprotiques, et contenant des fonctions thiols libres ou substituées, caractérisé en ce qu'il comprend, essentiellement, les étapes successives suivantes :
**a**_{**2**} solubilisation du collagène de départ dans au moins un solvant organique polaire,
**b**_{**2**} préparation de la sous-unité composé d'espacement-résidu cystéique comme défini dans la revendication 1 à fonctions thiols protégées,
**c**_{**2**} activation des fonctions carboxyles libres de la sous-unité,
**d**_{**2**} mise en réaction du collagène et de la sous-unité, de manière à obtenir un précurseur inerte du collagène modifié visé,
**e**_{**2**} activation directe du précurseur inerte, conduisant au collagène modifié porteur de fonctions thiols libres ou substituées, stabilisées.

10. Procédé d'obtention d'un collagène modifié, réticulable ou autoréticulable, soluble dans l'eau et/ou dans les solvants organiques polaires aprotiques, et contenant des fonctions thiols libres ou substituées, caractérisé en ce qu'il comprend, essentiellement, les étapes successives suivantes :
**a**_{**1**} solubilisation du collagène de départ dans au moins un solvant organique polaire aprotique,
**b**_{**1**} acylation et carboxylation du collagène solubilisé,
**c**_{**1**} activation des fonctions carboxyles libres du collagène,
**d**_{**1**} réaction du collagène activé avec un résidu cystéique comme défini dans la revendication 1 fonction(s) thiol(s) et, le cas échéant, à fonction(s) carboxylique(s) bloquée(s), de manière à obtenir un précurseur inerte du collagène modifié visé,
**e**_{**1**} activation indirecte du précurseur inerte, conduisant à du collagène réticulé par l'intermédiaire de ponts de disulfure intercaténaires,
**f**_{**1**} transformation du collagène réticulé en collagène modifié porteur de fonctions thiols libres ou substituées, stabilisées.

11. Procédé d'obtention d'un collagène modifié, réticulable ou autoréticulable, soluble dans l'eau et/ou dans les solvants organiques polaires aprotiques, et contenant des fonctions thiols libres ou substituées, caractérisé en ce qu'il comprend, essentiellement, les étapes successives suivantes :
**a**_{**2**} solubilisation du collagène de départ dans au moins un solvant organique polaire,
**b**_{**2**} préparation de la sous-unité composé d'espacement-résidu cystéique comme défini dans la revendication 1 à fonctions thiols protégées,
**c**_{**2**} activation des fonctions carboxyles libres de la sous-unité,
**d**_{**2**} mise en réaction du collagène et de la sous-unité, de manière à obtenir un précurseur inerte du collagène modifié visé,
**e**_{**2**} activation indirecte du précurseur inerte, conduisant à du collagène réticulé par l'intermédiaire de ponts de disulfure intercaténaires,
**f**_{**2**} transformation du collagène réticulé en collagène modifié porteur de fonction thiols libres ou substituées, stabilisées.

12. Procédé d'obtention d'un collagène réticulé insoluble, caractérisé en ce qu'il consiste à mettre en oeuvre les étapes **a**_{**1**} à **e**_{**1**} du procédé selon la revendication 8 ou les étapes **a**_{**2**} à **e**_{**2**} du procédé selon la revendication 9.

13. Produit intermédiaire obtenu lors de la mise en oeuvre du procédé selon l'une quelconque des revendications 8 à 12, caractérisé en ce qu'il est constitué par du collagène ayant réagi avec un diacide carboxylique, par l'intermédiaire d'au moins une partie de ses fonctions réactives OH et NH₂, et en ce qu'il comporte de 4 à 22 greffons carboxyliques pour 100 acides aminés.

14. Produit intermédiaire selon la revendication 13, caractérisé en ce que le diacide est l'acide succinique ou l'acide glutarique.

15. Application du collagène modifié, réticulable selon l'une quelconque des revendications 1 à 5, ou de celui obtenu par le procédé selon l'une quelconque des revendications 8 à 12, ou du collagène réticulé selon la revendication 6 ou 7, ou bien encore de celui obtenu par le procédé selon la revendication 12, à la préparation d'articles utilisables en médecine, du type implants, prothèses, peaux artificielles, pansements, revêtements de prothèses.

16. Application du collagène modifié, réticulable selon l'une quelconque des revendications 1 à 5, ou de celui obtenu par le procédé selon l'une quelconque des revendications 8 à 12, ou du collagène réticulé selon la revendication 6 ou 7, ou bien encore de celui obtenu par le procédé selon la revendication 12, pour la préparation d'articles et/ou de biomatériaux adhésifs et/ou de colles biologiques ou chirurgicales.

## Patentansprüche

1. Modifiziertes vernetzbares Kollagen, das in Wasser und/oder organischen polaren aprotischen Lösungsmitteln löslich ist und unsubstituierte oder substituierte Thiolgruppen aufweist, die von Cysteinresten getragen werden,
wobei die Reste:
- an das Kollagen zumindest teilweise über Spacerverbindungen gebunden sind
- und folgender allgemeinen Formel entsprechen: 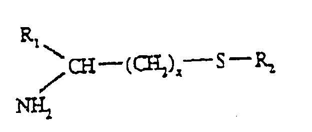 worin bedeuten:
- R₁ H oder COOR'₁, worin R'₁ bedeutet: eine aliphatische und/oder aromatische und/oder cyclische Kohlenwasserstoffkette;
- R₂ Wasserstoff oder eine aliphatische und/oder aromatische und/oder cyclische gegebenenfalls schwefelhaltige Kohlenwasserstoffkette;
- x 1 oder 2.

2. Modifiziertes Kollagen nach Anspruch 1, dadurch gekennzeichnet, daß die Spacerverbindung eine Kohlenwasserstoffeinheit mit Carboxygruppe ist.

3. Modifiziertes Kollagen nach Anspruch 2, dadurch gekennzeichnet, daß die Kohlenwasserstoffeinheit mit Carboxygruppe einer Dicarbonsäure abgeleitet ist, die befähigt ist, ein cyclisches Anhydrid zu bilden.

4. Modifiziertes Kollagen nach Anspruch 2 oder 3, dadurch gekennzeichnet, daß die Dicarbonsäure unter den folgenden Verbindungen ausgewählt ist: Bernsteinsäure, Glutarsäure, Phthalsäure, Itaconsäure, Citraconsäure und Maleinsäure sowie deren Derivaten.

5. Modifiziertes Kollagen nach Anspruch 1, dadurch gekennzeichnet, daß die Reste, welche die unsubstituierten oder substituierten Thiolgruppen tragen, unter den folgenden Verbindungen ausgewählt sind: Cystein, Cystin, Homocystein, Homocystin, Cysteamin und Cystamin.

6. Vernetztes unlösliches Kollagen, dadurch gekennzeichnet, daß die Brücken zwischen den Ketten zumindest teilweise aus Disulfidbrücken bestehen, welche von den in Anspruch 1 definierten Cysteinresten gebildet werden, die an das Kollagen zumindest teilweise über Spacerverbindungen gebunden sind.

7. Vernetzes Kollagen nach Anspruch 6, dadurch gekennzeichnet, daß es aus Kollagen nach einem der Ansprüche 1 bis 5 hergestellt ist.

8. Verfahren zur Herstellung eines modifizierten vernetzbaren oder selbstvernetzenden in Wasser und/oder organischen polaren aprotischen Lösungsmitteln löslichen Kollagens, das unsubstituierte oder substituierte Thiolgruppen aufweist, dadurch gekennzeichnet, daß es im wesentlichen aus den folgenden Schritten besteht:
a₁ Solubilisierung des Ausgangskollagens in mindestens einem organischen polaren aprotischen Lösungsmittel,
b₁ Acylierung und Carboxylierung des gelösten Kollagens,
c₁ Aktivierung der freien Carboxygruppen des Kollagens,
d₁ Umsetzung des aktivierten Kollagens mit einem in Anspruch 1 definierten Cysteinrest mit einer oder mehreren geschützten Thiolgruppen und gegebenenfalls einer oder mehreren geschützten Carbonsäuregruppen, um einen inerten Precursor des gewünschten modifizierten Kollagens herzustellen,
e₁ direkte Aktivierung des inerten Precursors, die zu dem modifizierten Kollagen führt, das unsubstituierte oder substituierte stabilisierte Thiolgruppen trägt.

9. Verfahren zur Herstellung eines modifizierten vernetzbaren oder selbstvernetzenden in Wasser und/oder organischen polaren aprotischen Lösungsmitteln löslichen Kollagen, das unsubstituierte oder substituierte Thiolgruppen aufweist, dadurch gekennzeichnet, daß es im wesentlichen aus folgenden Schritten besteht:
a₂ Solubilisierung des Ausgangskollagens in mindestens einem organischen polaren Lösungsmittel,
b₂ Herstellung der Untereinheit, die aus der Spacerverbindung und dem in Anspruch 1 definierten Cysteinrest mit geschützten Thiolgruppen besteht,
c₂ Aktivierung der freien Carboxygruppen der Untereinheit,
d₂ Umsetzung des Kollagens und der Untereinheit, um einen inerten Precursor des gewünschten modifizierten Kollagens herzustellen,
e₂ direkte Aktivierung des inerten Precursors, die zu dem modifizierten Kollagen führt, das unsubstituierte oder substituierte stabilisierte Thiolgruppen trägt.

10. Verfahren zur Herstellung eines modifizierten vernetzbaren oder selbstvernetzenden in Wasser und/oder organischen polaren aprotischen Lösungsmittel löslichen Kollagens, das unsubstituierte oder substituierte Thiolgruppen aufweist, dadurch gekennzeichnet, daß es im wesentlichen aus folgenden Schritten besteht:
a₁ Solubilisierung des Ausgangskollagens in mindestens einem organischen polaren aprotischen Lösungsmittel,
b₁ Acylierung und Carboxylierung des solubilisierten Kollagens,
c₁ Aktivierung der freien Carboxygruppen des Kollagens,
d₁ Umsetzung des aktivierten Kollagens mit einem in Anspruch 1 definierten Cysteinrest mit einer oder mehreren geschützten Thiolgruppen und gegebenenfalls einer oder mehreren geschützten Carboxygruppen, um einen inerten Precursor des gewünschten modifizierten Kollagens herzustellen,
e₁ indirekte Aktivierung des inerten Precursors, die zu Kollagen führt, das über Disulfidbrücken zwischen den Ketten vernetzt ist,
f₁ Umformung des vernetzten Kollagens in modifiziertes Kollagen, das unsubstituierte oder substituierte stabilisierte Thiolgruppen trägt.

11. Verfahren zur Herstellung eines modifizierten vernetzbaren oder selbstvernetzenden in Wasser und/oder organischen polaren aprotischen Lösungsmittel löslichen Kollagens, das unsubstituierte oder substituierte Thiolgruppen aufweist, dadurch gekennzeichnet, daß es im wesentlichen aus den folgenden Schritten besteht:
a₂ Solubilisierung des Ausgangskollagens in mindestens einem polaren organischen Lösungsmittel,
b₂ Herstellung der Untereinheit aus der Spacerverbindung und einem in Anspruch 1 definierten Cysteinrest mit geschützten Thiolgruppen,
c₂ Aktivierung der freien Carboxygruppen der Untereinheit,
d₂ Umsetzung des Kollagens und der Untereinheit, um einen inerten Precursor des gewünschten modifizierten Kollagens herzustellen,
e₂ indirekte Aktivierung des inerten Precursors, die zu Kollagen führt, das über Disulfidbrücken zwischen den Ketten vernetzt ist,
f₂ Umformung des vernetzten Kollagens in modifiziertes Kollagen, das freie oder substituierte stabilisierte Thiolgruppen trägt.

12. Verfahren zur Herstellung eines unlöslichen vernetzten Kollagens, dadurch gekennzeichnet, daß es darin besteht, die Schritte a₁ bis e₁ des Verfahrens nach Anspruch 8 oder die Schritte a₂ bis e₂ des Verfahrens nach Anspruch 9 durchzuführen.

13. Zwischenprodukt, das bei der Durchführung des Verfahrens nach einem der Ansprüche 8 bis 12 erhalten wird, dadurch gekennzeichnet, daß es aus Kollagen besteht, das mit einer Dicarbonsäure über mindestens einen Teil der reaktiven OH- und NH₂-Gruppen reagiert hat, und daß es 4 bis 22 Carbonsäurepfropfungen pro 100 Aminosäuren aufweist.

14. Zwischenprodukt nach Anspruch 13, dadurch gekennzeichnet, daß die Dicarbonsäure die Bernsteinsäure oder Glutarsäure ist.

15. Verwendung des modifizierten vernetzbaren Kollagens nach einem der Ansprüche 1 bis 5 oder des nach dem Verfahren nach einem der Ansprüche 8 bis 12 hergestellten Kollagens oder des vernetzten Kollagens nach Anspruch 6 oder 7 oder des nach dem Verfahren nach Anspruch 12 hergestellten Kollagens zur Herstellung von Gegenständen vom Typ der Implantate, Prothesen, künstlicher Haut, Verbände und Überzüge für Prothesen, die in der Medizin verwendbar sind.

16. Verwendung des modifizierten vernetzbaren Kollagens nach einem der Ansprüche 1 bis 5 oder des nach dem Verfahren nach einem der Ansprüche 8 bis 12 hergestellten Kollagens oder des vernetzten Kollagens nach Anspruch 6 oder 7 oder des nach dem Verfahren nach Anspruch 12 hergestellten Kollagens zur Herstellung von Gegenständen und/oder haftenden Biomaterialien und/ oder biologischen oder chirurgischen Haftmitteln.

## Claims

1. A cross-linkable modified collagen which is soluble in water and/or in aprotic polar organic solvents and which contains free or substituted thiol functions carried by cysteic residues,
said residues:
- being fixed to the collagen, at least some residues being fixed to the collagen via spacer compounds,
- and having the following general formula: 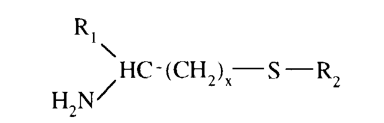
in which:
- R₁ = H, COOR'₁, with R'₁ = aliphatic and/or aromatic and/or cyclic carbon chain,
- R₂ is hydrogen or an aliphatic and/or aromatic and/or cyclic carbon chain which optionally contains sulphur.

2. The modified collagen according to claim 1, characterised in that the spacer compound is an hydrocarbon unit with carboxylic functions.

3. The modified collagen according to claim 2, characterised in that the hydrocarbon unit with carboxylic functions is derived from a dicarboxylic acid capable of forming a cyclic anhydride.

4. The modified collagen according to claim 2 or 3, characterised in that the dicarboxylic acid in question is selected from the group of following compounds: succinic, glutaric, phthalic, itaconic, citraconic and maleic acids, as well as derivatives thereof.

5. The modified collagen according to claim 1, characterised in that the residues carrying the free or substituted thiol functions are selected from the group of following compounds: cysteine, cystine, homocysteine, homocystine, cysteamine, cystamine.

6. An insoluble cross-linked collagen, characterised in that at least some of its intercatenary bridging structures are constituted of disulphide bridges formed from cysteic residues as defined in claim 1 and fixed to the collagen, at least some residues being fixed to the collagen via spacer compounds.

7. The cross-linked collagen according to claim 6, characterised in that it is obtained from the collagen according to any one of claims 1 to 5.

8. A method of obtaining a modified, cross-linkable or self-cross-linkable collagen which is soluble in water and/or in aprotic polar organic solvents and which contains free or substituted thiol functions, characterised in that it essentially comprises the following successive steps:
**a**_{**1**} solubilisation of the starting collagen in at least one aprotic polar organic solvent,
**b**_{**1**} acylation and carboxylation of the solubilised collagen,
**c**_{**1**} activation of the free carboxyl functions of the collagen,
**d**_{**1**} reaction of the activated collagen with a cysteic residue as defined in claim 1 and having thiol function(s), and, if need he, blocked carboxyl function(s), such that an inert precursor of the intended modified collagen is obtained, and
**e**_{**1**} direct activation of the inert precursor leading to the modified collagen carrying stabilised, free or substituted thiol functions.

9. A method of obtaining a modified, cross-linkable or self-cross-linkable collagen which is soluble in water and/or in aprotic polar organic solvents and which contains free or substituted thiol functions, characterised in that it essentially comprises the following successive steps:
**a**_{**2**} solubilisation of the starting collagen in at least one polar organic solvent,
**b**_{**2**} preparation of the sub-unit composed of spacer compound-cysteic residue as defined in claim 1 and, having protected thiol functions,
**c**_{**2**} activation of the free carboxyl functio ns of the sub-unit,
**d**_{**2**} reaction of the collagen with the sub-unit, such that an inert precursor of the intended modified collagen is obtained,and
**e**_{**2**} direct activation of the inert precursor, which leads to the modified collagen carrying stabilised, free or substituted thiol functions.

10. A method of obtaining a modified, cross-linkable or self-cross-linkable collagen which is soluble in water and/or in aprotic polar organic solvents and which contains free or substituted thiol functions, characterised in that it essentially comprises the following successive steps:
**a**_{**1**} solubilisation of the starting collagen in at least one aprotic polar organic solvent,
**b**_{**1**} acylation and carboxylation of the solubilised collagen,
**c**_{**1**} activation of the free carboxyl functions of the collagen,
**d**_{**1**} reaction of the activated collagen with a cysteic residue as defined in claim 1 and having thiol function(s), and, if need be, blocked carboxyl function(s), such that an inert precursor of the intended modified collagen is obtained,
**e**_{**1**} indirect activation of the inert precursor, leading to the collagen cross-linked via intercatenary disulphide bridges, and
**f**_{**1**} transformation of the cross-linked collagen into modified collagen carrying stabilised, free or substituted thiol functions.

11. A method of obtaining a modified, cross-linkable or self-cross-linkable collagen which is soluble in water and/or in aprotic polar organic solvents and which contains free or substituted thiol functions, characterised in that it essentially comprises the following successive steps:
**a**_{**2**} solubilisation of the starting collagen in at least one polar organic solvent,
**b**_{**2**} preparation of the sub-unit composed of spacer compound-cysteic residue as defined in claim 1 and, having protected thiol functions,
**c**_{**2**} activation of the free carboxyl functions of the sub-unit,
**d**_{**2**} reaction of the collagen with the sub-unit, such that an inert precursor of the intended modified collagen is obtained,
**e**_{**2**} indirect activation of the inert precursor, leading to the collagen cross-linked via intercatenary disulphide bridges, and
**f**_{**2**} transformation of the cross-linked collagen into modified collagen carrying stabilised, free or substituted thiol functions.

12. A method of obtaining an insoluble, cross-linked collagen, characterised in that it consists in carrying out steps a₁ to e₁ of the method according to claim 8, or steps a₂ to e₂ of the method according to claim 9.

13. An intermediate product obtained during the implementation of the method according to any one of claims 8 to 12, characterised in that it is constituted of collagen which has reacted with a carboxylic diacid, via at least some of its reactive OH and NH₂ functions, and in that it has from 4 to 22 carboxylic grafts per 100 amino acids.

14. An intermediate product according to claim 13, characterised in that the diacid is succinic acid or glutaric acid.

15. Application of the cross-linkable modified collagen according to any one of claims 1 to 5, or that obtained by the method according to any one of claims 8 to 12, or the cross-linked collagen according to claim 6 or 7, or even that obtained by the method according to claim 12, to the preparation of articles which can be used in medicine, of the types comprising implants, prostheses, artificial skin, dressings, coverings for prostheses, or the like.

16. Application of the cross-linkable modified collagen according to any one of claims 1 to 5, or that obtained by the method according to any one of claims 8 to 12, or the cross-linked collagen according to claim 6 or 7, or even that obtained by the method according to claim 12, for the preparation of adhesive articles and/or biomaterials and/or of biological or surgical glues.
